(19) Europäisches Patentamt
European Patent Office
Office européen des brevets

(11) **EP 1 997 817 B1**

(12) **EUROPEAN PATENT SPECIFICATION**

(45) Date of publication and mention
of the grant of the patent:
**19.04.2017 Bulletin 2017/16**

(21) Application number: **07736898.3**

(22) Date of filing: **16.03.2007**

(51) Int Cl.:
*C07D 317/38* (2006.01)     *B01D 9/02* (2006.01)

(86) International application number:
**PCT/JP2007/000241**

(87) International publication number:
**WO 2007/108213 (27.09.2007 Gazette 2007/39)**

(54) **PROCESS FOR PURIFYING ETHYLENE CARBONATE AND PROCESS FOR PRODUCING PURIFIED ETHYLENE CARBONATE**

VERFAHREN ZUR AUFREINIGUNG VON ETHYLENCARBONAT UND VERFAHREN ZUR HERSTELLUNG VON AUFGEREINIGTEM ETHYLENCARBONAT

PROCÉDÉ DE PURIFICATION DU CARBONATE D'ÉTHYLÈNE ET PROCÉDÉ DE PRODUCTION DE CARBONATE D'ÉTHYLÈNE PURIFIÉ

(84) Designated Contracting States:
**DE**

(30) Priority: **20.03.2006 JP 2006075953**

(43) Date of publication of application:
**03.12.2008 Bulletin 2008/49**

(60) Divisional application:
**11168915.4 / 2 374 803**

(73) Proprietors:
• **Mitsubishi Chemical Corporation**
  **Chiyoda-ku**
  **Tokyo 100-8251 (JP)**
• **Tsukishima Kikai Co., Ltd.**
  **Tokyo 104-0051 (JP)**

(72) Inventors:
• **YAMAGISHI, Masahiko**
  **Ibaraki, 806-004 (JP)**
• **FURUYA, Toshiyuki**
  **Ibaraki, 80-6004 (JP)**
• **SUDA, Hideki**
  **Tokyo; 1040051 (JP)**

(74) Representative: **Hoffmann Eitle**
**Patent- und Rechtsanwälte PartmbB**
**Arabellastraße 30**
**81925 München (DE)**

(56) References cited:
**GB-A- 1 086 028      JP-A- 07 089 905**
**JP-A- 09 227 550     JP-B1- 54 034 705**
**US-A- 4 519 875      US-A1- 2004 054 214**

EP 1 997 817 B1

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

TECHNICAL FIELD

The present invention relates to a process for purifying ethylene carbonate or a process for producing purified ethylene carbonate.

BACKGROUND ART

Cyclic carbonic esters such as ethylene carbonate, propylene carbonate and butylene carbonate are used as a solvent for a variety of polymer compounds, a reaction solvent for a variety of chemical reactions, an extractant, a blowing agent, a stabilizer for lubricating oils, etc. Ethylene carbonate is generally produced by reacting ethylene oxide with carbon dioxide at a high temperature and a high pressure. Thus, ethylene carbonate contains diols, such as ethylene glycol and diethylene glycol, derived from the raw materials. Ethylene carbonate also contains a trace amount of water in addition to the above impurities. Such water can react with ethylene carbonate to further yield the above-mentioned diols.

It is desirable that ethylene carbonate used as a solvent contain as small an amount of impurities as possible. In particular, it is described that ethylene carbonate having a low diol content is useful as an electrolytic solution (see Patent Document 1). Thus, it is necessary that impurities contained in ethylene carbonate should be removed. As a method for purifying ethylene carbonate, various methods such as a distillation method and a crystallization method are proposed.

Distillation is the most generally industrially practiced purification method. Since the boiling point of ethylene carbonate is as high as 246°C (at ambient pressure), however, purification of ethylene carbonate by distillation causes thermal deterioration of ethylene carbonate even when the distillation is performed under a reduced pressure. Namely, ethylene carbonate reacts with diols or water to form polymeric substances. Further, an investigation by the present inventors has revealed that since polymerized ethylene carbonate undergoes dissociation to yield diols, about 100 ppm of diols remain present in ethylene carbonate after distillation. Moreover, distillation must consume energy corresponding to the latent heat of evaporation of ethylene carbonate and must be performed at a high reflux ratio. As compared with crystallization which requires only cooling for the removal of sensible heat, therefore, distillation requires a large energy consumption.

Crystallization is a purification process in which a desired component is crystallized at a temperature at which impurity components are not crystallized and are prevented from contaminating the crystals of the desired component. Since the purification by crystallization consists of precipitation by cooling and dissolution by slight warming, deterioration by side reactions does not easily occur and energy consumption is low.

When purification of ethylene carbonate is actually performed by crystallization, however, impurities other than ethylene carbonate are occluded in the ethylene carbonate crystals during the crystallization (see Patent Document 2). Thus, in the method of Patent Document 2, crystals are allowed to form on walls of cooled vertical tubes. A part of the crystals thus formed is melted by warming and is allowed to flow downward for contact with crystals. The crystals with increased purity are scraped off. In the working example, ethylene carbonate with a purity of 98.8 to 99.84 % by weight is subjected to crystallization to obtain ethylene carbonate with a purity of 99.92 % by weight. Thus, the ethylene carbonate still contains 800 ppm of impurities such as glycols. Further, with this method, it is necessary to intermittently shift deposition and sweating of the crystals. The method also requires scraping of crystals.

A countercurrent contact method is known as a continuous crystallization process. In the countercurrent contact method, crystallization is carried out while contacting crystals of ethylene carbonate with liquid thereof for increasing the purity of ethylene carbonate. To be more specific, as the countercurrent contact method, there may be mentioned, for example, a method in which an upright column provided with a heating means at a bottom portion thereof and a cooling means at an upper portion thereof is used. When ethylene carbonate is purified by the above method, heated ethylene carbonate liquid flows upward while crystals thereof formed by cooling fall down so that they are brought into countercurrent contact with each other. The crystals are withdrawn as a melt from a bottom portion of the column, while the mother liquor is withdrawn from an upper portion thereof. When the above method is used for the purification of ethylene carbonate, however, the obtained ethylene carbonate has only a purity of 99.67 % by weight (Patent Document 3).

Patent Document 1: Japanese Patent Application Laid-Open (KOKAI) No. 10-270075
Patent Document 2: Japanese Patent Application Laid-Open (KOKAI) No. 07-89905
Patent Document 3: British Patent Specification No. 1,086,028

DISCLOSURE OF THE INVENTION

Problems to be Solved by the Invention:

The present invention has been made in view of the foregoing circumstance. It is an object of the present invention to provide a process for purifying ethylene carbonate or for producing purified ethylene carbonate, which has a significantly reduced content of, especially, diols.

Means for Solving the Problems:

The present inventors have made an earnest study. As a result, it has been found that operation conditions in the crystallization of ethylene carbonate play an important role in obtaining high purity ethylene carbonate and that ethylene carbonate having an extremely low impurity content can be obtained by conducting the crystallization process under specific conditions.

The present invention has been completed based on the above findings. The first aspect of the present invention resides in a process for purifying ethylene carbonate, comprising allowing crude ethylene carbonate crystals to fall from an upper portion of a tower, melting the crude ethylene carbonate crystals in a bottom portion of the tower, withdrawing a part of the obtained melt from the tower, and allowing the remainder of the obtained melt to flow upward as a reflux liquid for countercurrent contact with the falling crude ethylene carbonate crystals, characterized in that a solid-liquid countercurrent contact region maintained at a constant temperature is formed, said solid-liquid countercurrent contact region maintained at a constant temperature has a thickness of 0.75 to 40 m, and the crude ethylene carbonate crystals are contacted with the melt for 1.0 to 25.0 hours in said solid-liquid countercurrent contact region maintained at a constant temperature, wherein the solid-liquid countercurrent contact region has a temperature difference of 2.0°C or less in the vertical direction. The second aspect of the present invention resides in a process for producing purified ethylene carbonate in accordance with claim 1. The ethylene carbonate obtained by the above process may have a content of monoethylene glycol of 50 ppm by weight or less. The ethylene carbonate obtained by the above process may have a content of ethylene glycols of 50 ppm by weight or less.

Effect of the Invention:

By the process of the present invention, ethylene carbonate having a significantly reduced content of especially diols is provided. According to the process of the present invention, high purity ethylene carbonate can be continuously obtained in the form of a melt and, thus, it is no longer necessary to intermittently shift deposition and sweating of crystals and to conduct a crystal scraping operation.

BRIEF DESCRIPTION OF THE DRAWINGS

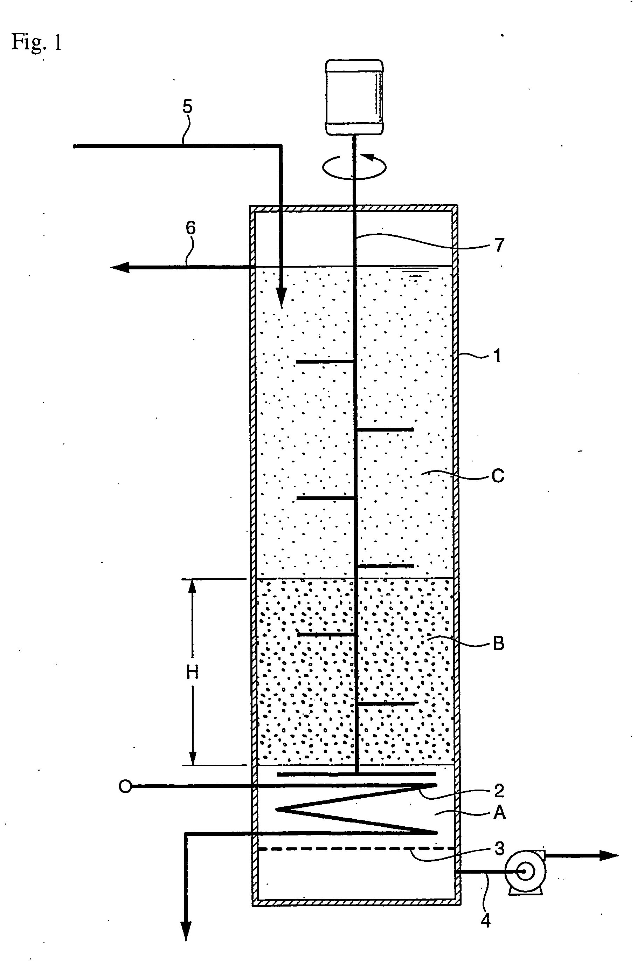

FIG. 1 is an explanatory view showing an example of a device for carrying out the process of the present invention. Explanation of Reference Numerals:

1:     Tower
2:     Heater
3:     Screen
4:     Product withdrawing pipe
5:     Crystal slurry feeding pipe
6:     Clarified liquid withdrawing pipe
7:     Stirring device

BEST MODE FOR CARRYING OUT THE INVENTION

Crude ethylene carbonate crystals used for the process of the present invention may be in any form as long as crude ethylene carbonate crystals are contained. For example, crude ethylene carbonate crystals themselves or a slurry containing crude ethylene carbonate crystals may be suitably used. Crude ethylene carbonate crystals used for the process of the present invention may be ethylene carbonate obtained as crystals by any known method or ethylene carbonate crystals obtained by crystallization of ethylene carbonate produced by any known method. (Hereinafter, "ethylene carbonate obtained as crystals" will not be differently treated from "ethylene carbonate crystals obtained by crystallization of ethylene carbonate". For the sake of convenience, "crude ethylene carbonate crystals" used for the process of the present invention will be referred to as "crude ethylene carbonate crystals" used for the process of the present invention or as "ethylene carbonate" used for the process of the present invention.) For example, ethylene carbonate obtained by reacting ethylene oxide with carbon dioxide at a high temperature and a high pressure may be used for the process of the present invention. When ethylene carbonate is produced by reacting ethylene oxide with carbon dioxide, the molar ratio of carbon dioxide to ethylene oxide is generally 0.1 to 5, preferably 0.5 to 3. The above reaction is generally carried out in the presence of a catalyst. Examples of the carbonation catalyst include a bromide or iodide of an alkali metal, a halide of an alkaline earth metal, an alkyl amine, a quaternary ammonium salt, an organic tin, germanium or tellurium compound and a quaternary phosphonium. The using amount of the catalyst is generally 1:1000 to 1:20 in terms of a molar ratio thereof to ethylene oxide. The above reaction generally yields ethylene carbonate with a purity of 95 to 99.5 % by weight and diols such as ethylene glycol and diethylene glycol.

As ethylene carbonate used for the process of the present invention, there may be used a process solution separated from an ethylene glycol production process using a halide of an alkaline earth metal or a quaternary phosphonium salt. To be more specific, there may be used ethylene carbonate which contains 25 % by weight or less of diols and 10 % by weight or less of water and which is obtained by subjecting a process solution, separated from an ethylene glycol production process, to a suitable concentration treatment.

In ethylene carbonate used for the process of the present invention, diols, such as ethylene glycol and diethylene glycol, derived from starting materials for the ethylene carbonate synthesis are contained. Such ethylene carbonate also contains a trace amount of water in addition to the above impurities. The water can react with ethylene carbonate to further yield diols. Ethylene carbonate used for the process of the present invention preferably has a high purity. However, ethylene carbonate having a purity of 50 % by weight or less may be used for the process of the present invention. Ethylene carbonate used for the process of the present invention may contain 10 % by weight or more of ethylene glycols (monoethylene glycol, diethylene glycol and triethylene glycol). Ethylene carbonate used for the process of the present invention may contain 10 % by weight or more of water.

In the present invention, ethylene carbonate as described above is subjected to a crystallization process using a countercurrent contact method to produce high purity ethylene carbonate. In the countercurrent contact method, crystallization is carried out while contacting crystals of ethylene carbonate with liquid thereof for increasing the purity of ethylene carbonate. As a specific example of the countercurrent contact method, there may be mentioned a method in which an upright column having a bottom portion provided with a heating means. To be more specific, there may be specifically mentioned a method which comprises allowing crude ethylene carbonate crystals to fall from an upper portion of a tower, melting the crude ethylene carbonate crystals in a bottom portion of the tower, withdrawing a part of the obtained melt from the tower, and allowing the remainder of the obtained melt to flow upward as a reflux liquid for countercurrent contact with the falling crude ethylene carbonate crystals. The above preferred process will be described in detail below. However, the crystallization process (countercurrent contact crystallization process) of the present invention may be embodied in other forms as long as they can attain the excellent effect of the present invention.

FIG. 1 is an explanatory view showing an example of a device for carrying out the production process of the present invention. In FIG. 1, designated as 1 is a tower which is generally maintained in a vertical position. The tower 1 has a bottom portion provided with a heater 2 through which a heating medium flows. A screen 3 and a product withdrawing pipe 4 are provided below the heater. At upper portions of the tower, a crystal slurry feeding pipe 5 and a clarified liquid withdrawing pipe 6 are provided. A stirring device 7 is disposed within the tower.

Crude ethylene carbonate crystals to be treated with the above device may be prepared by an arbitrary method, for example, by a method in which a solution containing ethylene carbonate or a slurry containing crude ethylene carbonate crystals is fed to an upright column provided with a cooling means at an upper portion thereof and is cooled to precipitate crystals. Alternatively, crude ethylene carbonate crystals used for the process of the present invention may be, for example, a crystal slurry obtained by subjecting ethylene carbonate to a method described below. The concentration of the crystals may be increased, if desired, by solid-liquid separation by using, for example, a liquid cyclone separator.

Japanese Patent Publication (KOKOKU) No. 04-31721 describes that a countercurrent, cooling crystallization and purification method for a melt was applied for the purification of p-dichlorobenzene containing o- and m-isomers as impurities. This method is not proposed for the purification of ethylene carbonate. The proposed method uses cooling type crystallization towers and a vertical melt purification tower in combination. The cooling type crystallization towers are each provided with a clarifying part at an upper part thereof. The vertical melt purification tower has a clarifying section at its upper portion and a heater at its lower portion and is operated at a temperature higher than the cooling type crystallization towers. Thus, in the proposed method, a crystal slurry obtained in the cooling type crystallization towers is fed to the clarifying section of the vertical melt purification tower that corresponds to the above-mentioned upright column. The crystals in the crystal slurry fed to the vertical melt purification tower are allowed to move downward to a crystal particle layer while being countercurrently contacted with an upwardly moving high purity mother liquor. In the crystal particle layer, the crystals slowly fall for countercurrently contacting with the upwardly moving reflux liquid, so that surfaces of the crystals are washed. The crystals which arrive at a melting section are melted. A part of the melt is withdrawn as a product, while the remainder of the melt moves upward as the flux liquid for washing the falling crystals. As a result of a series of the continuous operations, purification is achieved. The vertical melt purification tower is equipped with a stirring device for the purpose of stabilizing the movement of the crystals in a region beneath the clarifying section. In addition, as an improvement of the above method, there is also proposed a method in which an apparatus having an aging vessel which is provided between the cooling type crystallization towers and the vertical melt purification tower and which is operated at a temperature between the temperature of the cooling type crystallization towers and the temperature of the vertical melt purification tower (Japanese Patent Publication (KOKOKU) No. 06-91103).

In the device shown in FIG. 1, crude ethylene carbonate crystals are allowed to fall from an upper portion of the tower 1. The crude ethylene carbonate crystals are melted at a bottom portion of the tower. A part of the obtained melt is withdrawn from the tower 1, while the remainder of the obtained melt is allowed to flow upward as a reflux liquid for countercurrent contact with the falling crude ethylene carbonate crystals. Ethylene carbonate with a purity of 100 % by weight generally has a melting point (at ambient pressure) of 36.4°C. Monoethylene glycol has a melting point (at ambient pressure) of -12.6°C, while diethylene glycol has a melting point (at ambient pressure) of -6.5°C.

The heating temperature at the bottom portion of the tower 1 is not specifically limited as long as the temperature is not lower than the melting point of the ethylene carbonate withdrawn from the product withdrawing pipe 4. However, the lower the heating temperature, the more preferable. Accordingly, the heating temperature at the bottom portion of the tower 1 is generally 40 to 80°C, preferably 40 to 55°C. The reflux ratio (amount of residual liquid/amount of withdrawn liquid (weight ratio)) is generally 0.1 or more, preferably 0.4 or more, and is generally 5.0 or less, preferably 4.0 or less. A reflux ratio of not lower than the above lower limit is preferred for reasons of purity of the crystals. A reflux ratio of not greater than the above upper limit is preferred from the viewpoint of productivity.

The process of the present invention is characterized in that a sediment layer (B) of ethylene carbonate crystals is formed above the melt (A) within the tower 1, a temperature distribution of the sediment layer (B) in the vertical (thickness) direction being maintained within a constant range. Namely, the sediment layer (B) corresponds to "a solid-liquid countercurrent contact region maintained at a constant temperature". In the process of the present invention the sediment layer (B) of ethylene carbonate crystals is formed between a crystal particle layer (C) of the slowly falling crystals and the melt (A). In the present invention, the term "temperature distribution maintained within a constant range" and the term "maintained at a constant temperature" are intended to refer to the condition that a temperature difference is 2.0°C or less, preferably 1.0°C or less (taking a measurement error into consideration). The lesser the temperature difference the more preferable. Therefore, the lower limit of the temperature difference is 0.0°C.

Within the tower 1, the upwardly flowing melt is heat exchanged with the falling ethylene carbonate crystals through a countercurrent contact therebetween. In the conventional method, a temperature distribution was created throughout the entire mass within the tower 1 such that the temperature increases toward the bottom of the tower. In contrast, there is no such a temperature distribution in the sediment layer (B) of the ethylene carbonate crystals formed in the process of the present invention; namely the sediment layer is maintained at a constant temperature near the melting point of the ethylene carbonate.

In the sediment layer (B) of the ethylene carbonate crystals, a temperature distribution in the vertical (thickness) direction is maintained within a constant range. The filling density of the ethylene carbonate crystals is higher in the sediment layer (B) than that in the crystal particle layer (C) in which crystals slowly fall. The crystals are melted at the bottom of the sediment layer (B). A portion of the melt is countercurrently contacted with the crystals to wash the crystals. Yet, new crystals are sedimented on the upper part of the sediment layer. Crystallization is continuously performed under the conditions where these phenomena are repeatedly occurring. By the above operations, the ethylene carbonate crystals are highly purified.

The thickness (H) (absolute value) of the sediment layer (B) of ethylene carbonate crystals can be determined by measuring the temperature distribution in the tower 1 using multi-point thermometers. The thickness (H) of the sediment layer (B) is not specifically limited as long as a smooth countercurrent flow between the slurry and the reflux liquid is established. However, the lower limit of the thickness (H) is 0.75 m, preferably 1.00 m, while the upper limit is 40 m, preferably 20 m. When the thickness (H) of the sediment layer (B) is not less than the above lower limit, the period of time for which the crystals are contacted with the melt is prolonged, which is preferable from the viewpoint of purity of ethylene carbonate crystals obtained. When the thickness (H) of the sediment layer (B) is not greater than the above upper limit, on the other hand, an increase of the crystal density due to breakage of the crystals by their own weight can be prevented, which is preferable from the viewpoint of sufficient contact between the melt and the crystals. It is preferred that the ratio of the thickness (H) of the sediment layer (B) to the thickness of the tower 1 excluding the thickness of the melt (A) be 1:3 or more, more preferably 1:2 or more, from the viewpoint of suppressing a variation of the purity of ethylene carbonate which would be caused by a slight variation of the operating conditions (for example, variations in flow rates of the raw material slurry and of the product liquid withdrawn from the tower bottom). The upper limit of the ratio is preferably 9:10 for reasons of smooth flows of the slurry and reflux liquid.

The thickness (H) of the sediment layer (B) may be adjusted, for example, by the following methods. The thickness of the sediment layer (B) may be increased by reducing the amount of the product liquid withdrawn from the tower bottom or stopping the withdrawal of the product liquid; by reducing the amount of the ethylene carbonate crystals melted in the bottom portion of the tower (1) or stopping the melting of the crystals; by increasing the amount of the slurry fed from the upper portion of the tower (1); and by the like method. By performing reverse operations of the above, the thickness (H) of the sediment layer (B) may be reduced.

In the process shown in FIG. 1, it is preferred that stirring be carried out using a stirring device 7 since a variation of sedimentation distribution in the horizontal direction of the ethylene carbonate crystal sediment layer (B) can be reduced and since a deflected flow and a short pass of the upwardly moving reflux liquid do not easily occur. It is also preferred that the stirring device 7 be driven at a low revolution speed so that no turbulence occurs in the sediment layer (B) in the height direction thereof. To be more specific, the lower limit of a product of "revolution speed (rpm)" and "tower diameter (m)" is generally 0.1, preferably 0.2, while the upper limit thereof is generally 10.0, preferably 7.0, still more preferably 5.0.

In the process shown in FIG. 1, a "period of time for which the ethylene carbonate crystals are contacted with the melt in the solid-liquid countercurrent contact region maintained at a constant temperature" may be calculated, in terms of a residence time of the ethylene carbonate crystals in the sediment layer (B)" as follows:

$$\begin{aligned} &(\text{Residence time in the sediment layer (B))} \\ &= (\text{Thickness of the sediment layer}) \div (\text{Relative flow rate}) \\ &= (\text{Thickness of the sediment layer}) \div (\text{Descending speed of} \\ &\quad \text{the slurry} + \text{Ascending speed of the flux liquid}) \\ &= (\text{Thickness of the sediment layer}) \div \{(\text{Volume flow rate of} \\ &\quad \text{the slurry} + \text{Volume flow rate of the reflux liquid}) \div \\ &\quad (\text{Cross-sectional area of the tower})\} \\ &= (\text{Thickness of the sediment layer}) \div [\{(\text{Volume flow rate} \\ &\quad \text{of the product withdrawn}) \times (1 + \text{Reflux ratio}) + (\text{Volume} \\ &\quad \text{flow rate of the product withdrawn}) \div (\text{Reflux ratio})\} \div \\ &\quad (\text{Cross-sectional area of the tower})] \end{aligned}$$

In the above formulas, each flow rate is a superficial velocity.

The "period of time for which the ethylene carbonate crystals are contacted with the melt in the solid-liquid countercurrent contact region maintained at a constant temperature" may be controlled by the adjustment of the thickness (H) of the sediment layer (B) or the reflux ratio. The "period of time for which the ethylene carbonate crystals are contacted with the melt in the solid-liquid countercurrent contact region maintained at a constant temperature" is 1.0 hour or more, preferably 1.5 hours or more, more preferably 1.8 hours or more, from the viewpoint of the purity of the product. The upper limit is 25.0 hours from the viewpoint of the production efficiency.

In the present invention, ethylene carbonate having a monoethylene glycol content of 50 ppm by weight or less can be obtained. Further, in the present invention, ethylene carbonate preferably having a monoethylene glycol content of 20 ppm by weight or less can be obtained. Further, in the present invention, ethylene carbonate preferably having a content of ethylene glycols (monoethylene glycol, diethylene glycol and triethylene glycol) of 50 ppm by weight or less can be obtained. Ethylene carbonate having a low diol content is particularly preferably used as an electrolytic solution for a Li-ion battery. It is inferred that an oxygen-containing compound such as a diol reacts with a fluorine-containing electrolyte to gradually form fluoric acid which deteriorates recycle characteristics, electric capacitance and storage stability of the battery.

Analysis by the present inventors of impurities contained in conventionally known ethylene carbonate products has revealed that they contain the impurities shown in Table 2. In contrast, the present invention permits the obtainment of ethylene carbonate having very low contents of the impurities shown in Table 1. The present invention particularly preferably permits the obtainment of ethylene carbonate in which the content of each of the impurities shown in Table 2 is below the detection limit (5 ppm) of gas chromatography. Namely, the present invention is considered to be able to obtain an electrolytic solution which is particularly excellent as an electrolytic solution of an Li-ion battery.

The quantitative analysis of the impurities were carried out by gas chromatography under the conditions given below. The detection limit by the gas chromatography is 3 ppm by weight in the case of monoethylene glycol and about 5 ppm by weight in the case of diethylene glycol, triethylene glycol and the compounds shown in Table 2.

The analytic conditions for diethylene glycol were as follows. Column: VOCOL manufactured by SUPELCO Inc., Inside diameter: 0.53 mm, Length: 105 m, Membrane pressure: 3 $\mu$m, Column temperature: 60°C, Heating rate: 5°C/min, Final temperature: 200°C, Injection temperature: 120°C, Split ratio: 1, Detector: FID detector, Detector temperature: 220°C, Sample for analysis: diluted with acetonitrile to 1:1, and Sample amount: 1 $\mu$L.

The analytic conditions for other components (monoethylene glycol, etc.) than diethylene glycol were as follows. Column: INNOWAX manufactured by Hewlett Packard Corporation, Inside diameter: 0.53 mm, Length: 30 m, Membrane pressure: 1 $\mu$m, Column temperature: 45°C, Heating rate: 15°C/min, Final temperature: 200°C, Injection temperature: 130°C, Split ratio: 5, Detector: FID detector, Detector temperature: 200°C, Sample for analysis: diluted with acetonitrile by five-hold, and Sample amount: 1 $\mu$L.

<u>EXAMPLES</u>

The present invention will be further described in detail below by way of examples. It should be noted that the present invention is not limited to the following examples but may be embodied in other forms so far as they do not depart from the gist of the present invention.

Example 1

As a crystallization apparatus, a device similar to that shown in FIG. 1 was used. A tower 1 had an inside diameter of 200 mm and an effective height (height from an upper end of a melting section at the bottom of the tower to a top of the tower) of 2,000 mm and was provided inside with thermocouple thermometers arranged in the height direction at an interval of 25 cm. Through a heater 2 (heating coil), warm water (52.3°C at an inlet of the heating coil and 49.5°C at an outlet thereof; flow rate was 200 L/Hr) was passed as a heating medium. The calorific value supplied to the heating coil was 560 kcal·hr$^{-1}$ (= (flow rate) $\times$ (specific heat of water) $\times$ (temperature at the inlet of the coil - temperature at the outlet of the coil) = (200 L·hr$^{-1}$ $\times$ 1.00 kcal·L$^{-1}$·°C$^{-1}$ $\times$ (52.3-49.5)°C). A stirring device 7 used had a stirring shaft provided with horizontal stirring bars as stirring blades. The tower 1 was covered with a heat insulating material provided with a vertically extending slit-like window so that a state of the crystals was able to be checked with the naked eyes and the inside of the tower was able to be temporarily checked with the naked eyes.

An ethylene carbonate slurry (melted ethylene carbonate (EC): 47 % by weight, monoethylene glycol (MEG): 13 % by weight, diethylene glycol (DEG): 0.1 % by weight, water: 8 % by weight, crude ethylene carbonate crystals: 32 % by weight) previously obtained by a treatment in a cooling type crystallization vessel was supplied from a crystal slurry feeding pipe 5.

The crude ethylene carbonate crystals slowly fell and were melted at a bottom portion of the tower to form a melt. A portion of the melt flowed upward as a reflux liquid and was withdrawn from a clarified liquid withdrawing pipe 6 and supplied to a cooling type crystallization vessel (not shown). At this point in time, no EC was withdrawn from a product withdrawing pipe (4) so that a sediment layer of the crystals was allowed to form on the melt. The thickness of the crystal sediment layer reached 1,250 mm. While maintaining the sediment layer at that thickness, the operation was further continued for 8 hours so that the temperature distribution in the tower 1 was stabilized. Then, EC was begun to be withdrawn through the product withdrawing pipe 4. At this point in time, the feed rate of the raw material slurry was 450 kg/Hr while the withdrawing rate of EC was 6.9 kg/Hr. A reflux ratio was calculated to be 1.45 from the withdrawing rate of EC from the tower bottom and the melting amount of EC which was calculated from the calorific value supplied from the heating coil. Further, at this point in time, the residence time in the sediment layer was 1.9 hours. The stirring shaft was driven at a revolution speed of 10 rpm. A product of the "revolution speed (rpm)" and the "tower diameter (m)" was 2.0 (= 10 rpm $\times$ 0.20 m). The liquid density of EC was 1.31 kg·L$^{-1}$. The reflux ratio and the residence time in the sediment layer were calculated from the formulas shown below.

(Calorific value required for converting 1 kg of the crystals to a liquid at the coil temperature) = (Heat of fusion of EC) + (Temperature difference between the coil and the sediment layer) × (Specific heat of EC) = 27.3 kcal·kg$^{-1}$ + {(52.3°C + 49.5 °C)/2 − 36.4°C} × 0.41 kcal·kg$^{-1}$·°C$^{-1}$ = 33.2 kcal·kg$^{-1}$

(Amount of EC melted in the coil) = (Calorific value supplied to the heating coil) ÷ (Calorific value required for converting 1 kg of the crystals to a liquid at the coil temperature) = 560 kcal·hr$^{-1}$ ÷ 33.2 kcal·kg$^{-1}$ = 16.7 kg·hr$^{-1}$

(Reflux flow rate) = (Amount of EC melted in the coil) − (Withdrawing rate of EC) = 16.9 kg·hr$^{-1}$ - 6.9 kg·hr$^{-1}$ = 10.0 kg·hr$^{-1}$

(Reflux ratio) = (Reflux flow rate) ÷ (Withdrawing rate of EC) = 10.0 kg·hr$^{-1}$ ÷ 6.9 kg·hr$^{-1}$ = 1.45

(Descending speed of the slurry) = {(Withdrawing rate of EC) × (1 + Reflux ratio) ÷ (Liquid density of EC)} ÷ (Sectional area of the tower) = {6.9 kg·hr$^{-1}$ × (1 + 1.45) ÷ 1.31 kg·L$^{-1}$} ÷ (100 mm × 100 mm × 3.14) = 0.41 m·hr$^{-1}$

(Ascending speed of the flux liquid) = {(Withdrawing rate of EC) × (Reflux ratio) ÷ (Liquid density of EC)} ÷ (Sectional area of the tower) = (6.9 kg·hr$^{-1}$ × 1.45 ÷ 1.31 kg·L$^{-1}$) ÷ (100 mm × 100 mm × 3.14) = 0.24 m·hr$^{-1}$

$$\text{(Relative flow speed)} = \text{(Descending speed of the slurry)} + \text{(Ascending speed of the flux liquid)} = 0.41 \ \text{m} \cdot \text{hr}^{-1} + 0.24 \ \text{m} \cdot \text{hr}^{-1} = 0.65 \ \text{m} \cdot \text{hr}^{-1}$$

$$\text{(Residence time in the sediment layer)} = \text{(Height of the sediment layer)} \div \text{(Relative flow speed)} = 1.25 \ \text{m} \div 0.65 = 1.9 \ \text{hr}$$

The operation was further continued for 2 hours. It was confirmed with naked eyes and from temperature distribution that no turbulence occurred in the crystal sediment layer. The purity of EC withdrawn through the product withdrawing pipe 4 was measured by gas chromatography (detector: FID). As a result, the MEG content in the EC was found to be 5 ppm by weight and the content of the compounds in the EC shown in Table 2 was found to be below the detection limit (5 ppm by weight). Detailed data are shown in the Tables below. Incidentally, a study by the present inventors reveals that neither diethylene glycol nor triethylene glycol are detected in EC when the content of monoethylene glycol in the EC is several tens ppm. Accordingly, in the present case, the contents of diethylene glycol and triethylene glycol are considered to be each below the detection limit (5 ppm by weight).

Example 2

Purification of EC was carried out in the same manner as that in Example 1 except that the operation conditions were changed as summarized in Table 1. As a result, the MEG content in the EC was found to be 7 ppm by weight and the content of the compounds in the EC shown in Table 2 was found to be below the detection limit (5 ppm by weight). Detailed data are shown in the Tables below. Incidentally, a study by the present inventors reveals that neither diethylene glycol nor triethylene glycol are detected in EC when the content of monoethylene glycol in the EC is several tens ppm. Accordingly, in the present case, the contents of diethylene glycol and triethylene glycol are considered to be each below the detection limit (5 ppm by weight).

Comparative Example 1

The same device and the same raw material slurry as those in Example 1 were used. However, the device was operated in such a manner that no crystal sediment layer was formed. Thus, immediately after a melt of EC at 50°C had been formed, EC was withdrawn from the product withdrawing pipe 4. The feed rate of the raw material slurry and the withdrawing rate of EC through the product withdrawing pipe 4 were the same as those in Example 1. The purity of EC withdrawn through the product withdrawing pipe 4 was measured by gas chromatography (detector: FID). As a result, the MEG content in the EC was found to be 240 ppm by weight and the DEG content in the EC was found to be 2 ppm by weight. Incidentally, EC is highly hygroscopic in nature and absorbs moisture in the air. Thus, a moisture content of EC easily increases to several tens ppm during the measurement. For this reason, no measurement of the water content using a Karl Fischer measuring device was carried out. Instead, as described above, the purity of EC was evaluated in terms of the concentrations of MEG and DEG.

Comparative Example 2

Purification of EC was carried out in the same manner as that in Example 1 except that the operation conditions were changed as summarized in Table 1.

[Table 1]

| Conditions | Example 1 | Example 2 | Comparative Example 1 | Comparative Example 2 |
|---|---|---|---|---|
| Temperature of warm water at inlet | 52.3°C | 49.6°C | 54.2°C | 54.7°C |
| Temperature of warm water at outlet | 49.5°C | 47.6°C | 51.9°C | 52.0°C |
| Flow rate of warm water | 200 L·Hr$^{-1}$ | 1,200 L·Hr$^{-1}$ | 200 L·Hr$^{-1}$ | 200 L·Hr$^{-1}$ |

(continued)

| Conditions | Example 1 | Example 2 | Comparative Example 1 | Comparative Example 2 |
|---|---|---|---|---|
| Height of sediment layer | 1,250 mm | 1,000 mm | 250 mm | 500 mm |
| Product withdrawing rate | 6.9 kg·Hr⁻¹ | 6.5 kg·Hr⁻¹ | 6.7 kg·Hr⁻¹ | 6.1 kg·Hr⁻¹ |
| Revolution speed | 10 rpm | 10 rpm | 10 rpm | 10 rpm |
| Residence time in sediment layer | 1.9 Hr | 2.3 Hr | 0.52 Hr | 0.84 Hr |
| MEG content in product | 5 wt. ppm | 7 wt. ppm | 207 wt. ppm | 185 wt. ppm |

[Table 2]

Content of Oxygen-Containing Compounds Other Than Diols in

Ethylene Carbonate (EC)

Unit: ppm (proportion of GC peak area)

ND: not detected

| | | Example 1 | BASF EC-S | Huntsman High purity Grade |
|---|---|---|---|---|
| GC retention time (min) | Obtained (month and year) / Impurity (except diols) | – | Oct. 2004 | Sept. 2001 |
| 4.06 | | ND | ND | ND |
| 5.01 | | ND | ND | ND |
| 12.76 | | ND | 6 | ND |
| 13.38 | | ND | 8 | 3 |
| 16.03 | | ND | 3 | 5 |
| 17.50 | | ND | 28 | 12 |
| 20.97 | | ND | ND | ND |

[Table 2 continued]

| GC retention time (min) | Obtained (month and year) / Impurity (except diols) | Huntsman Jul. 2004 | Toa Gosei Mar. 2005 |
|---|---|---|---|
| 4.06 | (chemical structure) | 4 | ND |
| 5.01 | (chemical structure) | 7 | ND |
| 12.76 | (chemical structure) | 131 | ND |
| 13.38 | (chemical structure) | 25 | 5 |
| 16.03 | (chemical structure) | 26 | 14 |
| 17.50 | (chemical structure) | 7 | 12 |
| 20.97 | (chemical structure) | 19 | 34 |

**Claims**

1. A process for purifying ethylene carbonate or for producing purified ethylene carbonate, comprising allowing crude ethylene carbonate crystals to fall from an upper portion of a tower, melting the crude ethylene carbonate crystals in a bottom portion of the tower, withdrawing a part of the obtained melt from the tower, and allowing the remainder of the obtained melt to flow upward as a reflux liquid for countercurrent contact with the falling crude ethylene carbonate crystals, **characterized in that** a solid-liquid countercurrent contact region maintained at a constant temperature is formed,
said solid-liquid countercurrent contact region maintained at a constant temperature has a thickness of 0.75 to 40 m, and
the crude ethylene carbonate crystals are contacted with the melt for 1.0 to 25.0 hours in said solid-liquid countercurrent contact region maintained at a constant temperature, wherein the solid-liquid countercurrent contact region has a temperature difference of 2.0°C or less in the vertical direction.

2. The process for purifying ethylene carbonate or for producing purified ethylene carbonate according to claim 1, wherein the thickness of the solid-liquid countercurrent contact region having a constant temperature is determined by measuring the temperature distribution in the tower using multi-point thermometers and the period of time for which the ethylene carbonate crystals are contacted with the melt in the solid-liquid countercurrent contact region having a constant temperature is controlled by the adjustment of the above thickness or the reflux ratio.

3. The process for purifying ethylene carbonate or for producing purified ethylene carbonate according to claim 1 or 2, comprising reducing a content of monoethylene glycol contained in the ethylene carbonate to not more than 50 ppm by weight.

4. The process for purifying ethylene carbonate or for producing purified ethylene carbonate according to any one of claims 1 to 3, comprising reducing a content of ethylene glycols contained in the ethylene carbonate to not more than 50 ppm by weight.

5. The process for producing purified ethylene carbonate according to claim 1, wherein the process further comprises a step of reacting ethylene oxide with carbon dioxide to obtain the crude ethylene carbonate crystals.

**Patentansprüche**

1. Verfahren zur Reinigung von Ethylencarbonat oder zur Herstellung von gereinigtem Ethylencarbonat, wobei das Verfahren die folgenden Schritte umfasst:

   Fallenlassen von rohen Ethylencarbonatkristallen von einem oberen Teil eines Turms, Schmelzen der rohen Ethylencarbonatkristalle in einem Bodenteil des Turms, Abziehen eines Teils der erhaltenen Schmelze aus dem Turm und nach oben Strömenlassen des Rests der erhaltenen Schmelze als Rückflussflüssigkeit für den Gegenstromkontakt mit den fallenden rohen Ethylencarbonatkristallen,
   **dadurch gekennzeichnet, dass** ein auf einer konstanten Temperatur gehaltener Fest-flüssig-Gegenstromkontaktbereich gebildet wird,
   der auf einer konstanten Temperatur gehaltene Fest-flüssig-Gegenstromkontaktbereich eine Dicke von 0,75 bis 40 m hat und
   die rohen Ethylencarbonatkristalle 1,0 bis 25,0 Stunden lang mit der Schmelze im auf einer konstanten Temperatur gehaltenen Fest-flüssig-Gegenstromkontaktbereich in Kontakt gebracht werden, wobei der Fest-flüssig-Gegenstromkontaktbereich eine Temperaturdifferenz von 2,0°C oder weniger in der vertikalen Richtung aufweist.

2. Verfahren zur Reinigung von Ethylencarbonat oder zur Herstellung von gereinigtem Ethylencarbonat gemäß Anspruch 1, worin die Dicke des Fest-flüssig-Gegenstromkontaktbereichs mit einer konstanten Temperatur durch Messen der Temperaturverteilung im Turm unter Verwendung von Mehrpunktthermometern bestimmt wird und die Zeitspanne, für die die Ethylencarbonatkristalle mit der Schmelze im Fest-flüssig-Gegenstromkontaktbereich mit einer konstanten Temperatur kontaktiert werden, durch die Einstellung der obigen Dicke oder des Rückflussverhältnisses gesteuert wird.

3. Verfahren zur Reinigung von Ethylencarbonat oder zur Herstellung von gereinigtem Ethylencarbonat gemäß Anspruch 1 oder 2, umfassend das Reduzieren des Gehalts an im Ethylencarbonat enthaltenem Monoethylenglykol auf nicht mehr als 50 Gew.-ppm.

4. Verfahren zur Reinigung von Ethylencarbonat oder zur Herstellung von gereinigtem Ethylencarbonat gemäß einem der Ansprüche 1 bis 3, umfassend das Reduzieren des Gehalts an im Ethylencarbonat enthaltenen Ethylenglykolen auf nicht mehr als 50 Gew.-ppm.

5. Verfahren zur Herstellung von gereinigtem Ethylencarbonat gemäß Anspruch 1, wobei das Verfahren ferner einen Schritt des Umsetzens von Ethylenoxid mit Kohlendioxid umfasst, um die rohen Ethylencarbonatkristalle zu erhalten.

**Revendications**

1. Procédé de purification de carbonate d'éthylène ou de production de carbonate d'éthylène purifié, comprenant l'autorisation à des cristaux de carbonate d'éthylène bruts de chuter d'une partie supérieure d'une tour, la fusion des cristaux de carbonate d'éthylène bruts dans une partie inférieure de la tour, le retrait d'une partie du produit de fusion obtenue de la tour, et l'autorisation au reste du produit de fusion obtenu de s'écouler vers le haut sous la forme d'un liquide de reflux pour un contact à contre-courant avec les cristaux de carbonate d'éthylène bruts en chute, **caractérisé en ce qu'**une zone de contact à contre-courant solide-liquide maintenue à une température constante est formée,
   ladite zone de contact à contre-courant solide-liquide maintenue à une température constante a une épaisseur de 0,75 à 40 m, et
   les cristaux de carbonate d'éthylène bruts sont mis en contact avec le produit de fusion pendant 1 à 25 heures dans ladite zone de contact à contre-courant solide-liquide maintenue à une température constante, dans lequel la zone de contact à contre-courant solide-liquide a une différence de température de 2°C ou moins dans la direction verticale.

2. Procédé de purification de carbonate d'éthylène ou de production de carbonate d'éthylène purifié selon la revendication 1, dans lequel l'épaisseur de la zone de contact à contre-courant solide-liquide ayant une température constante est déterminée en mesurant la distribution de température dans la tour en utilisant des thermomètres multipoints, et le laps de temps pendant lequel les cristaux de carbonate d'éthylène sont mis en contact avec le produit de fusion dans la zone de contact à contre-courant solide-liquide ayant une température constante est contrôlé par l'ajustement de l'épaisseur ci-dessus ou le rapport de reflux.

3. Procédé de purification de carbonate d'éthylène ou de production de carbonate d'éthylène purifié selon la revendication 1 ou 2, comprenant la réduction d'une teneur en monoéthylène glycol contenu dans le carbonate d'éthylène à au plus 50 ppm en poids.

4. Procédé de purification de carbonate d'éthylène ou de production de carbonate d'éthylène purifié selon l'une quelconque des revendications 1 à 3, comprenant la réduction d'une teneur en éthylènes glycols contenus dans le carbonate d'éthylène à au plus 50 ppm en poids.

5. Procédé de production de carbonate d'éthylène purifié selon la revendication 1, dans lequel le procédé comprend en outre une étape consistant à faire réagir un oxyde d'éthylène avec du dioxyde de carbone pour obtenir les cristaux de carbonate d'éthylène bruts.

Fig. 1

**EP 1 997 817 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10270075 A
- JP 7089905 A
- GB 1086028 A
- JP 4031721 A
- JP 6091103 A